# EUROPEAN PATENT SPECIFICATION

(11) **EP 1 334 124 B1**
(45) Date of publication and mention of the grant of the patent: **03.01.2007**
(21) Application number: 01996557.3
(22) Date of filing: 07.11.2001
(51) Int. Cl.: C07K 14/435, C07K 7/08, A61P 31/04, A61P 17/00, A61P 35/00, A61K 38/10, A23L 3/3526, A23L 1/305

(54) **DIASTEREOMERIC PEPTIDES AND PHARMACEUTICAL COMPOSITIONS COMPRISING THEM**
DIASTEREOMERE PEPTIDE UND DIESE UMFASSENDE ARZNEIMITTEL
PEPTIDES DIASTEREOMERES ET COMPOSITIONS PHARMACEUTIQUES LES COMPRENANT

(30) Priority: 16.11.2000 IL 13972000
(43) Date of publication of application: 13.08.2003
(73) Proprietor: YEDA RESEARCH AND DEVELOPMENT CO. LTD., 76100 Rehovot (IL)
(72) Inventor: SHAI, Yechiel, 56333 Yahud (IL); OREN, Ziv, 75306 Rishon-Le-Zion (IL); SHAHAR, Michal, Holon 58342 (IL); EYAL, Nurit, Ness Ziona (IL)
(74) Representative: Vossius & Partner
(86) International application number: PCT/IL2001/001036
(87) International publication number: WO 2002/040529

(56) References cited:
- WO-A-97/31019
- WO-A-98/37090
- Z OREN & Y SHAI: "Cyclization of a cytolytic amphipatic alpha-helical peptide and its diastereomer: effect on structure, interaction with model membranes, and biological functions" BIOCHEMISTRY., vol. 39, no. 20, April 2000 (2000-04), pages 6103-6114, XP002223235 AMERICAN CHEMICAL SOCIETY. EASTON, PA., US ISSN: 0006-2960
- Y HONG ET AL.: "Structure and organization of hemolytic and nonhemolytic diastereomers of antimicrobial peptides in membranes" BIOCHEMISTRY., vol. 38, no. 51, 1999, pages 16963-16973, XP002223236 AMERICAN CHEMICAL SOCIETY. EASTON, PA., US ISSN: 0006-2960
- Z OREN ET AL.: "A repertoire of novel antibacterial diastereomeric peptides with selective cytolytic activity" JOURNAL OF BIOLOGICAL CHEMISTRY., vol. 272, no. 23, 1997, pages 14643-14649, XP002071936 AMERICAN SOCIETY OF BIOLOGICAL CHEMISTS, BALTIMORE, MD., US ISSN: 0021-9258

## Description

The present invention relates to diastereomeric peptides and to pharmaceutical compositions comprising them.

Antimicrobial resistance to conventional antibiotics has become a maj or problem worldwide because of their extensive use. In fact, strains of bacteria already exist that are resistant to all available antibiotics.

The development of new families of antibiotics that can overcome the resistance problem has become a very important task. One such major family consists of the antimicrobial peptides. Antimicrobial peptides are natural antibiotics that constitute a major part of the innate immunity of a wide range of organisms. During the past two decades, numerous studies have demonstrated the essential role of antimicrobial peptides as the first line of defense against invading pathogens and their uncontrolled proliferation.

An important property of most antimicrobial peptides is their ability to selectively kill bacteria but not normal eukaryotic cells. Many studies indicated that a specific secondary structure is required and that the common feature found in most of them are an amphipathic character and a net positive charge. Most of the studies were carried out with linear amphipathic α-helical cytolytic peptides (≤ 40 amino acids) from a host-defense system (Boman, 1995). These polypeptides vary considerably in chain length, hydrophobicity, and overall distribution of charges, but share a common structure upon association with lipid bilayers, namely, an amphipathic α-helical structure. This group includes: (i) lytic peptides that are toxic only to microorganisms, e.g., cecropins isolated from the cecropia moth and magainins and dermaseptins isolated from the skin of frogs; (ii) lytic peptides that lyse mammalian cells, such as δ-hemolysin isolated from S. aureus; and (iii) non-cell-selective lytic peptides such as the bee venom melittin and the neurotoxin pardaxin, that can lyse microorganisms and mammalian cells.

Both pardaxin and melittin are endowed with potent antibacterial activity on Gram-negative and Gram-positive bacteria; however, they also exhibit high hemolytic activity towards human erythrocytes. Recently, we have incorporated several D-amino acids into the α-helical cytolytic peptides pardaxin (Shai and Oren, 1996; International PCT Publication No. WO 97/31019) and melittin (Oren and Shai, 1997; WO 97/31019). Although the resulting diastereomeric analogues of pardaxin and melittin had reduced cytotoxic effects on mammalian cells, they retained high antibacterial activity; thus providing a basis for designing novel peptide antibiotics composed of D-and L-amino acids that are selective to microorganisms.

International PCT Publication WO 98/37090 of the same applicants describes peptides comprising both L- and D-amino acid residues with a net positive charge greater than +1, having a sequence of amino acid residues such that a corresponding amino acid sequence comprising only L-amino acid residues is not found in nature. These non-natural synthetic peptides may be composed of varying ratios of at least one hydrophobic amino acid such as, for example, leucine, isoleucine, glycine, alanine, valine, phenylalanine, proline, tyrosine and tryptophan, and at least one positively charged amino acid such as, for example, lysine, arginine and histidine, in which sequence at least one of the amino acid residues is a D-amino acid. Several diastereomers comprising from 6 to 30 amino acid residues, conferring a cytolytic activity on microbial pathogenic organisms and malignant cells and are non-hemolytic are disclosed in WO 98/37090, but the biological activity was tested only for some 6-mer, 8-mer and 12-mer peptides. Some short-model peptides (12-amino acid long) composed of only leucine and lysine at varying ratios, in which one-third of the sequence consisted of D-amino acids, were further investigated and some of them were found to have a selective antimicrobial activity and reduced hemolytic activity (Hong, Oren & Shai, 1999; Oren, Hong & Shai, 1997).

It is an object of the present invention to provide non-natural synthetic diastereomeric peptides that are suitable for topical application against several pathogens.

It is another object of the present invention to provide non-natural synthetic diastereomeric peptides that are suitable for treatment of cancer.

It has now been found that certain diastereomeric peptides not disclosed in any of the references cited above, and in particular not disclosed in the above-mentioned WO 98/37090, have an enhanced activity for topical application and for the treatment of cancer in comparison to the closest diastereomer disclosed in said WO 98/37090.

The present invention thus relates to a diastereomeric peptide with a net positive charge greater than +1, and having at least 15 amino acid residues, wherein said residues are selected from: (i) leucine and lysine; (ii) leucine, lysine and arginine; (iii) the residues of(i) or (ii) and glycine, serine, aspargine, threonine, or glutamine at the N-terminus; or (iv) the residues of (i), (ii) or (iii) and lysine, arginine, glycine, or serine at the C-terminus; and (v) cyclic analogues of (i), (ii), (iii) or (iv). In one embodiment, the diastereomeric peptide of the invention is composed solely of leucine and lysine residues. Examples of such peptides are the 15-mer peptides herein designated Peptides **2, 3, 4, 7** and **10,** of the sequences:
**2** Lys-Leu-**Leu**-Leu-Lys-Leu-Leu-**Lys-Lys**-Leu-Leu-Lys-**Leu**-Leu-Lys-NH₂
**3** Leu-Lys-**Leu**-Leu-Lys-Lys-Leu-**Leu-Lys**-Lys-Leu-Leu-**Lys**-Leu-Leu-NH₂
**4** Leu-Lys-**Leu**-Leu-Lys-**Lys**-Leu-**Leu-Lys**-Lys-Leu-Leu-**Lys**-Leu-Leu-NH₂
**7** Lys-Leu-**Leu**-Lys-Leu-Leu-**Lys-Lys**-Leu-**Leu**-Lys-Leu-**Leu**-Leu-Lys-NH₂
**10** Lys-Leu-**Leu**-Leu-Lys-**Leu**-Leu-**Lys-Lys**-Leu-Leu-Lys-**Leu**-Leu-Lys-NH₂

In another embodiment, the diastereomeric peptide of the invention is composed of leucine, lysine and arginine residues. Examples of such peptides are the 15-mer peptides herein designated Peptides **6** and **9,** of the sequences:
**6** Lys-Leu-**Leu**-Leu-Arg-**Leu**-Leu-**Lys-Lys**-Leu-Leu-Arg-**Leu**-Leu-Lys-NH₂
**9** Lys-Leu-**Leu**-Arg-Leu-Leu-**Lys-Lys**-Leu-**Leu**-Arg-Leu-**Leu**-Leu-Lys-NH₂

In a further embodiment, the invention relates to such diastereomeric peptides composed of leucine and lysine or of leucine, lysine and arginine residues, and having at the N-terminus an additional amino acid selected from glycine, serine, aspargine, threonine, or glutamine. In one particular embodiment this additional amino acid is glycine residue, and examples thereof are the 16-mer peptides of the sequences:
**5** Gly-Leu-**Leu**-Leu-Arg-**Leu**-Leu-**Lys-Lys**-Leu-Leu-Arg-**Leu**-Leu-Lys-Lys-NH₂
**8** Gly-Leu-**Leu**-Lys-Leu-Leu-**Lys-Lys**-Leu-**Leu**-Lys-Leu-**Leu**-Leu-Lys-Lys-NH₂

In still another embodiment, the invention relates to such diastereomeric peptides having at the C-terminus an amino acid selected from lysine, arginine, glycine, and serine.

In still a further embodiment, the invention relates to cyclic analogues of the diastereomeric peptide. Examples of such cyclic peptides include the peptides of the sequences:

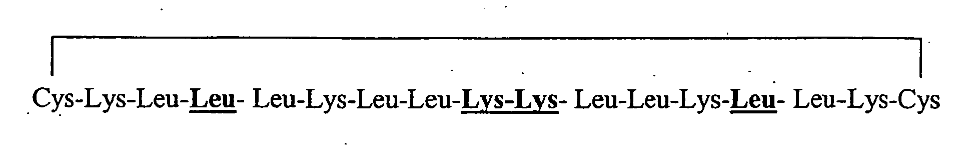
Cys-Lys-Leu-Leu- Leu-Lys-Leu-Leu-Lys-Lys- Leu-Leu-Lys-Leu- Leu-Lys-Cys

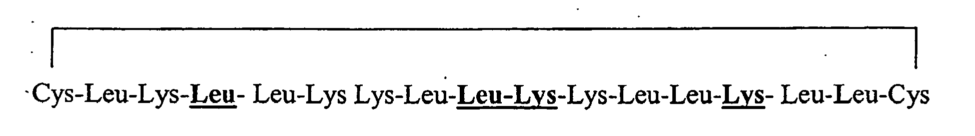
Cys-Leu-Lys-Leu- Leu-Lys Lys-Leu-Leu-Lys-Lys-Leu-Leu-Lys- Leu-Leu-Cys

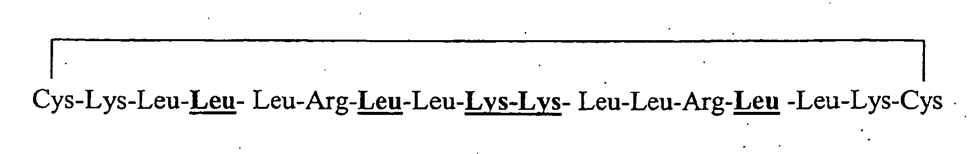
Cys-Lys-Leu-Leu- Leu-Arg-Leu-Leu-Lys-Lys- Leu-Leu-Arg-Leu -Leu-Lys-Cys

In another aspect, the present-invention provides a pharmaceutical composition comprising a diastereomeric peptide of the invention and a pharmaceutically acceptable carrier.

In one embodiment, the invention provides pharmaceutical compositions for topical application, for example for the treatment of topical infections caused by pathogenic organisms such as bacterial infections, particularly infections caused by bacteria resistant to antibiotics, and infections caused by pathogenic fungi. Although all peptides of the invention are contemplated for topical use as antifungal and antibacterial, it is shown hereinafter that the peptides herein designated peptides **2-8** are good candidates for these purposes. In addition, the peptides **2** and **6** were shown to be effective against antibiotic-resistant bacteria.

In another embodiment, the present invention provides pharmaceutical compositions comprising a diastereomeric peptide of the invention for the treatment of cancer. It is contemplated that all peptides of the invention are useful for the treatment of malignant tumors as shown herein for peptides **2, 8, 9** and **10.** In particular, peptides **9** and **10** were shown in experiments in vitro and in vivo to be effective against melanoma, basal and squamous cell carcinomas, or breast, colon, lung, and prostate tumors.

The diastereomeric peptides of the invention are effective against mycoplasma and can further be used to control/eliminate mycoplasma infection in cell cultures in a method comprising treating the cell culture with said diastereomeric peptide.

The invention further relates to the use of a diastereomeric peptide of the invention for the preparation of a pharmaceutical composition for topical application or of a pharmaceutical composition for the treatment of cancer.

The invention still further relates to the use of a diastereomeric peptide of the invention for the preparation of a pharmaceutical composition for topical application for the treatment of an infection caused by a pathogenic organism. In another embodiment, the invention relates to the use of a diastereomeric peptide of the invention for the preparation of a pharmaceutical composition for the treatment of a malignant tumor.
**Fig**. **1** is a graph showing tumor growth curve in SCID mice containing human breast MCF-7 cells after administration of peptide **9** of the invention (squares), mitomycin (triangles) and PBS (control, circles).
**Fig. 2** is a graph showing body weight curve of SCID mice containing human breast MCF-7 cells after administration of peptide **9** of the invention (squares), mitomycin (triangles) and PBS (control, circles).
**Fig. 3** is a graph showing lung weight of C-57 black mice infected with lung carcinoma D-122 cells after administration of peptide **9** of the invention or PBS (control).

The diastereomeric peptides of the invention are cytolytic agents of very low toxicity as evaluated herein in animal models. The results of the skin and eye irritation studies revealed low toxicity of the peptides at concentrations considerably higher than those necessary for their antibacterial, anticancer and antifungal activities.

In order to improve the cytolytic activity of the diastereomeric peptide, the effect of several important parameters such as length, amphipathic organization, the location and number of D-amino acids, additional amino acid residues at the N- and C-termini, and polarity of the diastereomeric peptides on their potency, selectivity and spectrum of activity were examined. For this purpose we synthesized and structurally and functionally characterized a series of linear cytolytic diastereomers. The peptides were then characterized with regard to their biological activity towards pathogenic cells such as bacteria, cancer cells and fungi, on the one hand, and normal mammalian cells such as NIH-3T3 normal fibroblasts cell line, on the other.

The potency and selectivity of the novel diastereomers of the invention is demonstrated herein in the anticancer, antifungal and antimycoplasma assays. The group of 15- and 16-mer diastereomers shown herein was significantly more active than the prior art 12-mer diastereomer (see peptide **1** in Example 1) against malignant cells, pathogenic fungi and mycoplasma. Furthermore, they were active against the malignant cells at concentrations that are 3-15 lower than the concentrations at which they act against NIH-3T3 normal fibroblasts cells.

In one embodiment of the present invention, the new diastereomeric peptides of the invention are particularly useful for topical application. As used herein, the term "topical" means "pertaining to a particular surface area" and the topical agent applied to a certain area of said surface will affect only the area to which it is applied. Thus any and all applications in which the peptides act locally and not through the blood circulation are encompassed by the present invention.

The high potency, the wide spectrum of activity and the low in vivo toxicity of the model diastereomers of the invention pave the way for their use in topical applications against a wide variety of pathogenic organisms in topical infections. Such applications include, but are not limited to, treatment of acne, fungal infections of the scalp, fungal or bacterial infections related to surgical or traumatic wounds, chronic or poorly healing skin lesions (especially in diabetics), vaginal infection (vaginitis), eye and ear infections and burn wounds, infections of mouth and throat, and localized infections such as chronic pulmonary infections in cystic fibrosis, emphysema or asthma that can be treated with aerosol or other formulation for nasal or pulmonary application.

Topical infections are characterized by opportunistic colonization of a wide range of endogenous and exogenous pathogenic cells. In addition, the treatment of severe wounds such as bums or poorly healing wounds e.g. foot ulcers in diabetes mellitus patients, require long term administration of antibiotics and lead to selection of resistant bacteria such as *Streptococcus pyogenes* or the methicilin-resistant *Staphylococcus aureus.* These problems could be overcome by the diastereomeric peptides of the invention due to their wide spectrum of activity and their ability to act against non-resistant and resistant bacteria as demonstrated in the case of peptide no. 2 against *Staphylococcus aureus* and the methicilin-resistant *Staphylococcus aureus.* The observed resistance of the diastereomers to proteolytic digestion may enable them to reach the digestive system in intact form and to eliminate there bacterial infections such as chronic gastric mucosal infestation by *Helicobacter pylori* and intestinal bacterial infections.

The activity of the diastereomeric peptides against different strains of fungi indicate their potential use for the treatment of nail fungus such as: (i) onychomycoses, the most current nail infection caused mainly by dermatophytes, in particular by *Trichophyton rubrum,* and less frequently by *Trichophyton mentagrophytes* and *Epidermophyton floccosum;* (ii) infections caused by mold; and (iii) infections caused by yeasts, particularly *Candida albicans,* as in chronic paronychia and onycholysis, and chronic mucocutaneous candidosis.

In another embodiment of the invention, the new diastereomeric peptides of the invention are useful as anticancer agents. The observed high potency of the diastereomeric peptides against a variety of malignant cells as shown in the examples herein indicate the existence of a common target for their action. This target is most probably the malignant cell membrane, that-has been shown to express higher levels of negatively charged phosphatidylserine than normal mammalian cells (Utsugi *et al.,* 1991). Thus, the diastereomeric peptide may also act against topical and skin cancers such as melanoma, against secondary topical tumors in breast, lung, prostate, and colon cancer patients, as well as against basal and squamous cell carcinomas.

The diastereomers of the invention may also be used for food preservation, as food supplements in veterinary compositions, as alternative to antibiotics for animal nutrition, as anti-mycoplasma, antibacterial, and antifungal agents for tissue culture media, and as reagents for tiansformation/transfection of target cells with desired DNA or RNA molecules.

In summary, the simple model diastereomeric peptides of the invention provide an efficient alternative to the complex sequence of hydrophobic and polar amino acids of known native amphipathic α-helical antimicrobial peptides, required to maintain their monomeric form, selectivity and interface location. Furthermore, by modulating the length, amphipathicity, location and number of D-amino acids and the polarity of the hydrophobic and positively charged amino acids, the membrane selectivity and antipathogenic activity of the desired diastereomeric peptides can be determined. Thus, the increased structural and sequence flexibility of the model diastereomeric antimicrobial peptides of the invention provide important advantages for the design of a repertoire of potent antipathogenic diastereomeric polypeptides for the treatment of diseases.

For topical application, the active components can be formulated with a variety of cosmetically and/or pharmaceutically acceptable carriers. The term "pharmaceutically acceptable carrier" refers to a vehicle which delivers the active components to the intended target and which will not cause harm to humans or other recipient organisms. As used herein, "pharmaceutical" will be understood to encompass both human and animal pharmaceuticals. Useful carriers include, for example, water, acetone, ethanol, ethylene glycol, propylene glycol, butane-1, 3-diol, isopropyl myristate, isopropyl palmitate, or mineral oil. Methodology and components for formulation of pharmaceutical compositions are well known, and can be found, for example, in Remington's Pharmaceutical Sciences, Eighteenth Edition, A. R. Gennaro, Ed., Mack Publishing Co. Easton Pà., 1990. The carrier may be in any form appropriate to the mode of delivery, for example, solutions, colloidal dispersions, emulsions (oil-in-water or water-in-oil), suspensions, creams, lotions, gels, foams, mousses, sprays and the like.

The formulation, in addition to the carrier and the antimicrobial components, also can comprise other optional materials which may be chosen depending on the carrier and/or the intended use of the formulation. Additional components include, but are not limited to, antioxidants, chelating agents, emulsion stabilizers, e.g. carbomer, preservatives, e.g. methyl paraben, fragrances, humectants, e.g. glycerine, waterproofing agents, e.g. PVP/Eicosene Copolymer, water soluble film-formers, e.g. hydroxypropyl methylcellulose, oil-soluble film formers, cationic or anionic polymers, and the like.

The antimicrobial components are well-suited for combination with other active components intended for topical application.

The invention will now be described with reference to some non-limiting examples.

### EXAMPLES

### Experimental procedures

### (i). Materials

4-Methyl benzhydrylamine resin (BHA) and butyloxycarbonyl (Boc) amino acids were purchased from Calbiochem-Novabiochem Co. (La Jolla, CA, USA). Other reagents used for peptide synthesis included trifluoroacetic acid (TFA, Sigma), N,N-diisopropylethylamine (DIEA, Sigma), dicyclohexylcarbodiimide (DCC, Fluka), 1-hydroxybenzotriazole (1-HOBT, Pierce), and dimethylformamide (DMF, peptide synthesis grade, Biolab, IL). All other reagents were of analytical grade. Buffers were prepared in double-distilled water.

### (ii) Peptide Synthesis and Purification

Peptides were synthesized by a solid phase method on 4-methyl benzhydrylamine resin (BHA) (0.05 meq) (Merrifield *et. al.,* 1982; Shai *et. al.,* 1990). The resin-bound peptides were cleaved from the resin by hydrogen fluoride (HF) and, after HF evaporation; and washing with dry ether, extracted with 50% acetonitrile/water. HF cleavage of the peptides bound to BHA resin resulted in C-terminus amidated peptides. Each crude peptide contained one major peak, as revealed by RP-HPLC (reverse phase high-performance liquid chromatography), that was 60-80% pure peptide by weight. The synthesized peptides were further purified by RP-HPLC on a C₁₈ reverse phase Bio-Rad semi-preparative column (250 x 10 mm, 300 nm pore size, 5-µm particle size). The column was eluted in 40 min, using a linear gradient of 25-60% acetonitrile in water, both containing 0.05 % TFA (v/v), at a flow rate of 1.8 ml/min. The purified peptides, which were shown to be homogeneous (~95%) by analytical HPLC, were subjected to amino acid analysis and electrospray mass spectroscopy to confirm their composition and molecular weight.

### (iii) Synthesis of the cyclic diastereomers.

The cyclic peptides are synthesized by a solid-phase method as described in section (ii) above, without or with cysteine residues at both the N- and C-termini of the peptides. The cyclization without cystein is carried out by protecting the N-terminal, activating the C-terminal, deprotection of the N-terminal and reaction of the C- and N-terminal groups while still bound to the resin. When the peptide contains cystein residues at both the N- and C- termini, after HF cleavage and RP-HPLC purification, the peptides are solubilized at low concentration in PBS (pH 7.3), and cyclization is completed after 12 h. The cyclic peptides are further purified on RP-HPLC and subjected to amino acid analysis to confirm their composition, and SDS-PAGE to confirm their monomeric state.

### Example 1. Synthesis of 15-mer and 16-mer linear diastereomeric peptides and cyclic peptides

The following 15-mer and 16-mer C-amidated diastereomeric peptides **2-10** composed of the the hydrophobic amino acid Leu and the positively charged amino acid Lys and, optionally, the positively charged amino acid Arg and/or the N-cap amino acid Gly, and containing 4-5 D-amino acid residues, were synthesized as described in Experimental Procedures, sections (ii) and (iii). Peptide **1** is a 12-mer diastereomer described in the above-mentioned WO 98/37090 and herein used for comparison purposes. The peptides will be represented hereinafter by numerals in bold.

The bold and underlined amino acids are D-amino acids.
**1**. [D]-L^{3,4,8,10}-K₄L₈ of the sequence:
   Lys-Leu-**Leu-Leu**-Lys-Leu-Leu-**Leu**-Lys-**Leu**-Leu-Lys-NH₂
**2**. [D]-L^{3,13}K^{8,9}-K₆L₉ of the sequence:
   Lys-Leu-**Leu**-Leu-Lys-Leu-Leu-**Lys-Lys**-Leu-Leu-Lys-**Leu**-Leu-Lys-NH₂
**3**. [D]-L^{3,8},K^{9,13}-K₆L₉ of the sequence:
   Leu-Lys-**Leu**-Leu-Lys Lys-Leu-**Leu-Lys**-Lys-Leu-Leu-**Lys**-Leu-Leu-NH₂
**4.** [D]-L^{3,8},K^{6,9,13}-K₆L₉ of the sequence:
   Leu-Lys-**Leu**-Leu-Lys **Lys**-Leu-**Leu-Lys**-Lys-Leu-Leu-**Lys**-Leu-Leu-NH₂
**5.** [D]-L^{3,6,13},K^{8,9}-GK₄R₂L₉ of the sequence:
   Gly-Leu-**Leu**-Leu-Arg-**Leu**-Leu-**Lys-Lys**-Leu-Leu-Arg-**Leu**-Leu-Lys-Lys-NH₂
**6**. [D]-L3,6,13,K8,9-K₄R₂L₉ of the sequence:
   Lys-Leu-**Leu**-Leu-Arg-**Leu**-Leu-**Lys-Lys**-Leu-Leu-Arg-**Leu**-Leu-Lys-NH₂
**7.** [D]-L3,10,13,K7,8- K₆L₉ of the sequence:
   Lys-Leu-**Leu**-Lys-Leu-Leu-**Lys-Lys**-Leu-**Leu**-Lys-Leu-**Leu**-Leu-Lys-NH₂
**8**. [D]-L^{3,10,13},K^{7,8}-GK₆L₉ of the sequence:
   Gly-Leu-**Leu**-Lys-Leu-Leu-**Lys-Lys**-Leu-**Leu**-Lys-Leu-**Leu**-Leu-Lys-Lys-NH₂
**9**. [D]-L^{3,10,13,}K^{7,8}-K₄R₂L₉ of the sequence:
   Lys-Leu-**Leu**-Arg-Leu-Leu-**Lys-Lys**-Leu-**Leu**-Arg-Leu-**Leu**-Leu-Lys-NH₂
**10.** [D]-L^{3,6,13},K^{8,9,13}-K₆L₉ of the sequence:
   Lys- Leu-**Leu**-Leu-Lys-**Leu**-Leu-**Lys-Lys**-Leu-Leu-Lys-**Leu-**Leu-Lys-NH₂

   The following cyclic amidated diastereomeric peptides **11-13** were prepared:
**11**. Cyclic- [D]-L3,13,K8,9-K₆L₉ Cys-Lys-Leu-Leu-Leu-Lys-Leu-Leu-Lys-Lys-Leu-Leu-Lys-Leu-Leu-Lys-Cys
**12.** Cyclic- [D]-L^{3,8},K^{9,13}-K₆L₉ 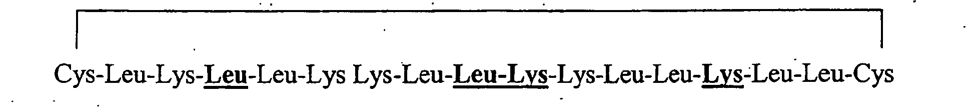
Cys-Leu-Lys-Leu-Leu-Lys Lys-Leu-Leu-Lys-Lys-Leu-Leu-Lys-Leu-Leu-Cys
**13.** Cyclic- [D]-L^{3,6,13},K^{8,9}-K₄R₂L₉

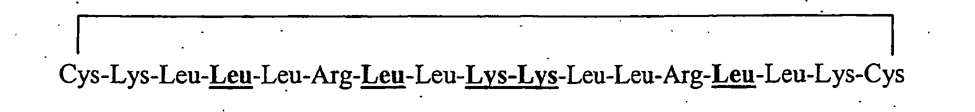

Cys-Lys-Leu-Leu-Leu-Arg-Leu-Leu-Lys-Lys-Leu-Leu-Arg-Leu-Leu-Lys-Cys

### Example 2. Antibacterial activity of the diastereomeric peptides

The antibacterial activity of the peptides was examined in sterile 96-well plates (Nunc F96 microtiter plates) in a final volume of 100 µl as follows: Aliquots (50 µl) of a suspension containing bacteria at concentration of 1 X 10⁶ Colony-Forming Units (CFU)/ml in culture LB (Lauria broth) medium were added to 50 µl of water containing the peptide in serial 2-fold dilutions in water. Inhibition of growth was determined by measuring the absorbance at 600 nm with a Microplate autoreader E1309 (Bio-tek Instruments), after an incubation time of 18-20 h at 37°C. Antibacterial activities were expressed as the minimal inhibitory concentration (MIC), the concentration at which 100% inhibition of growth was observed after 18-20 h of incubation. The bacteria used were: *Escherichia coli* ATCC 25922, *Acinetobacter baumannii* ATCC 19606, *Pseudomonas aeruginosa* ATCC 27853, *Staphylococcus aureus* ATCC 6538P, *Enterococcus faecalis* ATCC 29212, *Enterobacter cloacae* ATCC 49141. The antibacterial activity of peptides **2,** and **6** was also examined against resistant bacteria: methicilin-resistant *Staphylococcus aureus* (MRSA) ATCC 700698 and vancomycin-resistant *Enterococcus faecium* (VRE) ATCC 700221.

The results for peptides **1-8** summarized in **Table 1,** reveal that peptides **2-8** of the invention are significantly more potent than known peptide **1** against most bacteria examined. In addition, peptides **2** and **6** are highly active against the above-mentioned resistant bacteria MRSA and VRE, indicating that these bacteria are not resistant to the diastereomeric peptides.

**Table 1**

| **Minimal Inhibitory Concentration (µg/ml) of diastereomeric peptides for bacteria growth** | | | | | | | | |
|---|---|---|---|---|---|---|---|---|
| **Peptide Designat ion** | **E. coli** | **P. aeruginosa** | **A. baumannii** | **E. cloacae** | **E. faecalis** | **S. aureus** | **S. aureus (MRSA)** | **E. faecium (VRE)** |
| **1** | 22 | 14 | . 31 | 16 | 100 | 28 | N.D. | N.D. |
| **2** | 3.37 | 6.75 | 5 | 9 | 12.5 | 12.5 | 12.5 | 3.37 |
| **3** | 1.5 | 5 | 19 | 9 | 4 | 48 | N.D. | N.D. |
| **4** | 2.9 | 5 | 23 | 11 | 18 | 54 | N.D. | N.D. |
| **5** | 1.2 | 5 | 4 | 6 | 43 | 11 | N.D. | N.D. |
| **6** | 6.75 | 6.75 | 15 | 6.5 | 50 | 18 | 50 | 6.75 |
| **7** | 2.5 | 6 | 4 | 10 | 121 | 10 | N.D. | N.D. |
| **8** | 2.1 | 6 | 5 | 8 | 120 | 7 | N.D. | N.D. |

| | | | | | | | | |
|---|---|---|---|---|---|---|---|---|
| N.D. = Not determined | | | | | | | | |

### Example 3. Antifungal activity of the diastereomers

The antifungal activity of the diastereomers was examined in sterile 96-well plates (Nunc F96 microtiter plates) in a final volume of 200 µL as follows: 100 µl of a suspension containing fungi at concentration of 1 x 10⁴ Colony-Forming Units (CFU)/ml in culture medium (RPMI 1640, 0.165M MOPS pH 7.0, with L-glutamine, without NaHCO₃) was added to 100 µl of water containing the peptide in serial 2-fold dilutions in water. The fungi were incubated in the presence of the diastereomeric peptides for 24-48 h at 35°C in a Binder KB115 incubator under agitation. Growth inhibition was determined by measuring the absorbance at 620 nm with a Microplate autoreader E1309 (Bio-tek Instruments). Antifungal activity is expressed as the minimal inhibitory concentration (MIC), the concentration at which 100 % inhibition of growth was observed after the incubation time mentioned above. The fungi used were: *Aspergillus niger* ATCC 9642, *Candida albicans* ATCC 10231 and *Cryptococuus neoformans* ATCC 66031.

The results for peptides **1-8,** summarized in **Table 2**, show that while the known 12-mer peptide **1** was inactive against *C. albicans* and *A. niger,* peptides of the invention **2-8** were active against all the fungi examined.

**Table 2**

| **MIC (µg/ml) of the diastereomeric peptide for fungi** | | | |
|---|---|---|---|
| **Peptide Designation** | **C *albicans*** | ***C. neoformans*** | ***A. niger*** |
| | **ATCC 10231** | **ATCC 66031** | **ATCC 9642** |
| **1** | >200 | 4.7 | >200 |
| **2** | 100 | 1.4 | 50 |
| **3** | 40 | 2.8 | 90 |
| **4** | 50 | 5.6 | 180 |
| **5** | 50 | 3 | 50 |
| **6** | 150 | 1.5 | 200 |
| **7** | 50 | 2.9 | 50 |
| **8** | 30 | 1.5 | 30 |

### Example 4. Anticancer activity of the diastereomeric peptides

### 4a. In vitro activity against mouse melanoma cell line

The anticancer activity of the diastereomers **1, 2, 8, 9** and **10** was examined against B16 F10 mouse melanoma cell line. Melanoma cells were grown in RPMI-1640 medium supplemented with 10% fetal calf serum and antibiotics, at 37°C, in humidified atmosphere at 5% CO₂ and 95% air. In addition, the cell-selectivity of the diastereomeric peptides was studied by examining their effect on NIH-3T3 normal fibroblasts cell line. NIH-3T3 mouse fibroblasts cells were grown in DMEM medium supplemented with 10% bovine calf serum and antibiotics under the same conditions as described above for B16 F10 mouse melanoma cells. Melanoma cells (2x10⁵ cell ml⁻¹) or NIH-3T3 cells (2.5x10⁵ cell ml-¹) were seeded in tissue culture plates (3.5 cm). After 48 h, cells were washed twice with the corresponding medium. Then the cells were incubated at 37°C in medium (without serum and antibiotics) in the presence of the tested diastereomeric peptides dissolved in PBS, at different concentrations. In control experiments, PBS alone or the chemotherapeutic agent Mitomycin C were added to the cells. Following 24 h of incubation, the cells were harvested with trypsin (0.25%)-EDTA (0.05%) and centrifuged for 10 min at 270 x g. The precipitated cells were suspended in the suitable medium and counted in a hemozitometer (Neubauer). Cell viability was determined by Trypan blue dye exclusion. Anticancer activities were expressed as LC50, the concentration at which 50% cell mortality was observed after 24 h incubation.

The results for peptides **2, 8, 9,** and **10,** summarized in **Table 3,** show that the diastereomers **2, 8, 9, 10** of the invention are significantly more potent than the known 12-mer diastereomer 1 against the melanoma cell line. Furthermore, peptides **2, 8, 9, 10** were active against melanoma cell line at concentrations which are 3-15 fold lower than the concentration at which they act against NIH-3T3 normal fibroblast cell line. In contrast, both mitomycin C and peptide **1** were active against both cell lines at similar concentration. These results clearly reveal that the new diastereomeric peptides of the invention are more selective than the clinically used chemotherapeutic agent mitomycin C.

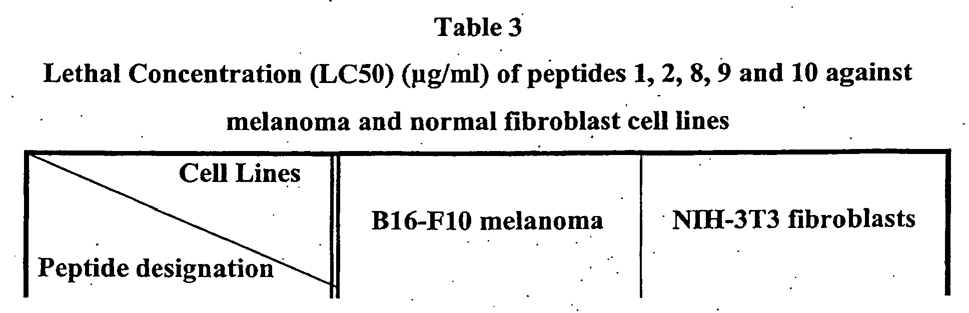

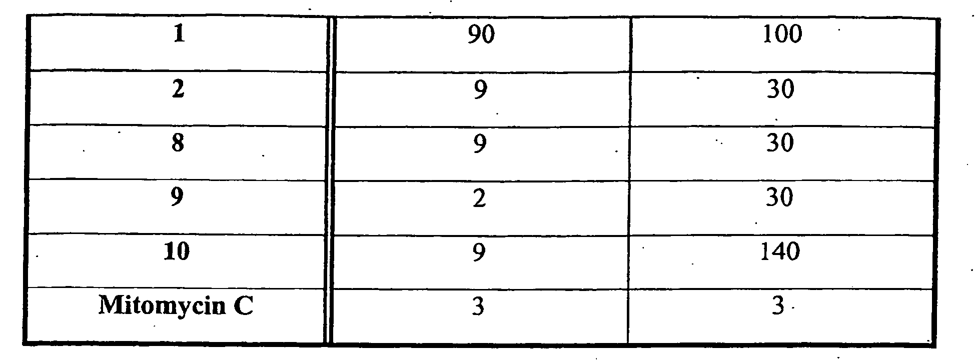

### 4b. In vitro activity against human tumor cell lines

The anticancer activity of diastereomers **9** and **10** of the invention was also examined against 5 different human tumor cell lines as shown in Table 4 below:

**Table 4**

| **Human tumor cell lines** | | |
|---|---|---|
| **Cell** | **Source** | **Type of Cell Line** |
| **MCF-7** | ATCC HTB-22 | Breast adenocarcinoma, pleural effusion, human |
| **HT-29** | ATCC HTB-38 | Adenocarcinoma, colon, moderately well-differentiated grade II, human |
| **A 549** | ATCC CCL-185 | Lung carcinoma, human |
| **SK-MEL-** | ATCC HTB-70 | Malignant melanoma, human |
| **PC-3** | ATCC CRL- | Prostate, adenocarcinoma, human |

Aliquots of 100 µl of tumor cell suspension (about 2.5-5 x 10³/well) were placed in 96-well microtiter plates in an atmosphere of 5% CO₂ at 37°C. After 24 hours, 100 µl of growth medium, 2 µl of test solution; mitomycin or vehicle (PBS, pH=7.4) were added per well in duplicate for an additional 72-hour incubation. The tested diastereomeric peptide was evaluated at concentrations of 100, 10, 1, 0.1 and 0.01 µM. At the end of incubation, 20 µl of AlamarBlue 75% reagent was added to each well for another 6-hour incubation before detection of cell viability by fluorescence intensity. Fluorescence intensity was measured using a Spectraflour Plus plate reader with excitation at 530 nm and emission at 590 nm.

The assays were used to detect changes in cell proliferation based on the ability of viable cells to cause AlamarBlue to change from its oxidized (non-fluorescent, blue) to a reduced (fluorescent, red) form. With the results obtained from the AlamarBlue reaction, cell proliferation can be quantified and metabolic activity of viable cells can be examined.

As can be seen in **Table 5,** the diastereomers **9** and **10** exhibit significant growth inhibition (>50%) relative to the respective vehicle-treated control group at assay concentrations between about 1 and 20 µM in all of the 5 different human tumor cell lines. This shows that the diastereomeric peptides of the invention have a wide spectrum of activity against cancer. The potent activity of the peptides against different types of tumor cell lines and their cell selectivity make them good candidates to be used as anticancer drugs.

**Table 5**

| **IC50 (µg/ml) of peptides 9 and 10 against human tumor cell lines** | | |
|---|---|---|
| **Cell line** | **Test** | ***IC₅₀** |
| **Tumor, Breast, MCF-7** | 10 | 3.6 µM |
| | 9 | 3.3 µM |
| **Tumor, Colon, HT-29** | 10 | 15 µM |
| | 9 | 2.2 µM |
| **Tumor, Lung, A549** | 10 | 2.4 µM |
| | 9 | 1.4 µM |
| **Tumor, Melanoma, SK-** | 10 | 2.6 µM |
| **MEL-5** | 9 | 1.2 µM |
| **Tumor, Prostate, PC-3** | 10 | 8.9 µM |
| | 9 | -1.6 µM |

| | | |
|---|---|---|
| *IC₅₀ (50% Inhibition Concentration) is the test compound concentration in which the increase from time₀ of the number of treated cells was only 50% as compared to corresponding increase in the vehicle-(control) treated cells at the end of the experiment. | | |

### 4c. In vivo activity against breast cancer

The in-vivo anticancer assay was conducted on SCID (Severe- Combined Immune Deficiency) mice using the xenograft model of human breast MCF-7 cells. Groups of 6 SCID female mice weighing 18-20 g (6-week old), bred in an animal isolator (IVC racks) under specific pathogen-free (SPF) conditions at 24 ± 1°C were used. Viable human breast carcinoma MCF-7 cells (ATCC HTB-22- 1 x 10⁷ cells in 0.2 ml of PBS) were injected subcutaneously (s.c.) into the dorsal side of SCID mice. 50 µg/mouse of estradiol benzoate was injected s.c. weekly as supplement for 4 weeks. When tumor growth reached ≥ 5 mm in diameter (about day 12), the animals were randomly assigned into groups of six and administration of vehicle and/or test compounds was started. Peptide **9** at 3 mg/kg or vehicle control (PBS, pH =7.4) in a dosing volume 10 ml/kg was administrated intravenously (IV) to animals twice a week for 6 doses: Mitomycin at a dose of 2 mg/kg was administrated intraperitoneally (IP) twice a week for 5 doses. The tumor size, body weight and the signs of overt animal toxicity after each treatment were recorded and observed. At the end of the experiment, hematological analysis was carried out.

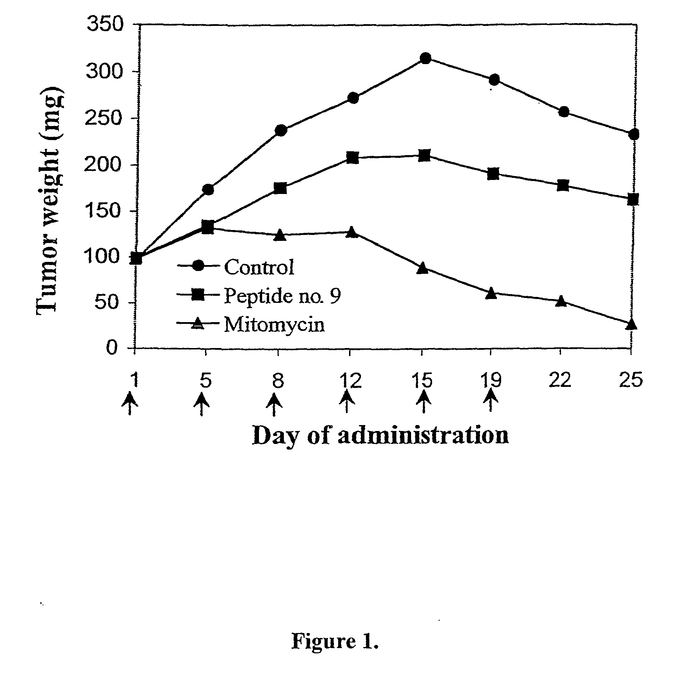

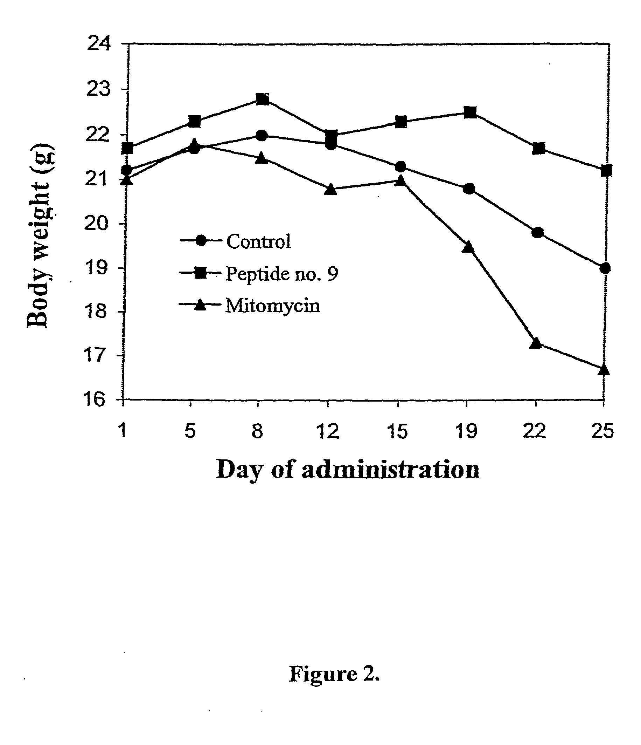

The results summarized in **Fig. 1** show that peptide **9** at dosage of 3 mg/kg exhibited inhibitory effect upon the tumor growth over the test period. Although the chemotherapeutic agent mitomycin was more potent than peptide **9 (Fig. 1**), the animals treated with mitomycin exhibited severe side effects throughout the assay period, whereas the animals treated with peptide **9** displayed side effects which appeared only after the compound administration and disappeared within **1** hour. **Fig. 2** shows that the animals treated with mitomycin and even the control mice showed signs of weakness and loss of weight that aggravated as the experiment proceeded. In contrast, the animals treated with peptide no. **9** were in good condition, they did not express any signs of weakness and they maintained their body weight throughout the study period. In addition, hematological analysis that was conducted at the end of the assay revealed that mitomycin also caused a significant reduction on white blood cells (WBC), red blood cells (RBC) and platelets while peptide **9** did not cause a reduction in WBC and platelets and only a slight reduction in RBC was noted in animals treated with peptide **9 (Table 6)**.

The results obtained in this experiment indicate that peptide 9 is a potential candidate-for-use as anticancer drug, probably in higher concentrations than shown herein. In comparison to mitomycin C, peptide 9 was found to be less toxic and to cause only mild temporary side effects, whereas mitomycin C caused severe side effects that persisted for a long period, even few weeks after its last administration.

**Table 6**

| **Hematology Analysis** | | | | | | |
|---|---|---|---|---|---|---|
| **Compound** | **Route** | **Dose** | **N** | **Hematology analysis on Day 25, MEAN ± SEM** | | |
| | | | | **WBC (x 10³/µl)** | **RBC (x 10⁶/µl)** | **PLT (x 10³/µl)** |
| **Control** | IV | 10 ml/kg x 6 | 6 | 2.95 ± 0.39 | 11.92 ± 0.12 | 1302 ± 52 |
| **Peptide No. 9** | IV | 3 mg/kg x 6 | 6 | 2.22 ± 0.22 | 11.18 ± 0.11 | 1310 ± 55. |
| **Mitomycin** | IP | 2 mg/kg x 6 | 6 | 0.67 ± 0.16 | 10.55 ± 0.37 | 396 ± 80 |

| | | | | | | |
|---|---|---|---|---|---|---|
| N = No. of animals. WBC = White blood cells; RBC = Red blood cells; PLT = Platelets. | | | | | | |

### 4d. In vivo activity against lung cancer

In order to test the capacity of the diastereomers of the invention to inhibit lung metastasis formation, the murine malignant 3LL D-122 Lewis lung carcinoma model (D122 clone of the 3LL carcinoma of C57BL/6 origin) was used with C57 black male mice (Porgador et al., 1993). The animals were bred in an animal isolator (IVC racks) under specific pathogen-free (SPF) conditions at 24 ± 1⁰C. Malignant 3LL D-122 cells (1 x 10⁶ cells/0.2 ml PBS) were injected intravenously (i.v.) to twenty 8-9 week-old C57 black male mice weighing 18-22 g. After 24 hours, the animals were randomly assigned into two groups and administration of vehicle (control) or of a test compound was started. Peptide **9** at 5 mg/kg or control (PBS, pH = 7.4) at 10 ml/kg was administered i.v. every day to the mice for three days in the first week, and then once a week in the next two weeks, for a total of 5 treatments in period of 21 days. On day 28, mice were sacrificed and their lungs were removed and weighed in order to measure the extent of lung metastasis. The metastatic load is defmed as the mean lung weight of mice infected with D-122 cells minus the mean lung weight of 5 normal mice (158 ± 2 mg).

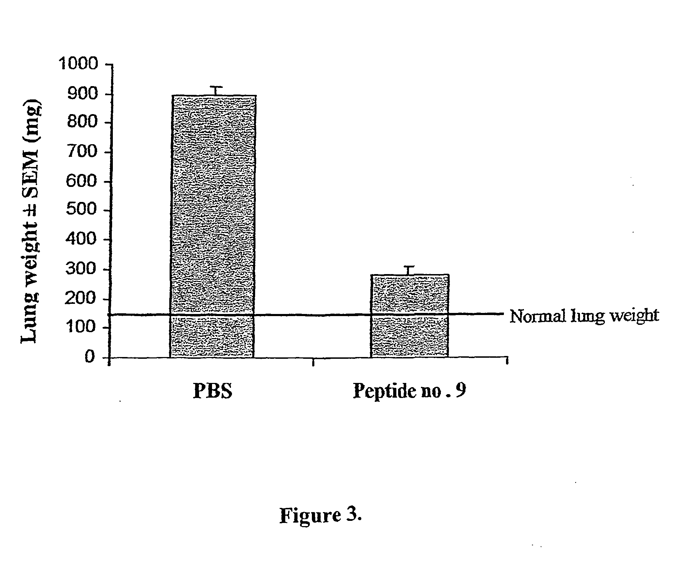

The results are depicted in **Fig. 3.** As shown, mice infected with D-122 lung carcinoma cells and treated with peptide **9** of the invention exhibited significantly lower lungs weight (282 ± 28 mg) in comparison with D-122 infected and untreated mice (896 ± 39 mg). The metastatic load was decreased by 80% in the group treated with peptide **9** compared to the untreated group. These findings reveal that peptide **9** is also significantly active against lung tumor metastases and can be used as a broad spectrum anticancer drug.

### Example 5. Resistance of the diastereomers to proteolytic digestion

In order to reach their target, the diastereomers have to withstand proteolytic digestion of proteases which may occur during the time of their administration site till they reach the target site. The susceptibility of the diastereomer **2** of the invention to proteolytic digestion by pepsin (from porcine stomach mucosa, Sigma), trypsin (from bovine pancreas, Sigma), and elastase (from human leukocytes, Sigma) was assessed by reverse-phase HPLC. As a control we used the native, all L-amino acid antimicrobial peptide cecropin B. Equal amounts of the peptides were dissolved in PBS (35 mM phosphate buffer/0.15 M NaCl, pH 7.3) at final concentration of 140 □M to which 25 □M of protease were added. The samples were incubated under agitation for 30 min at 37°C. After the addition of the appropriate protease inhibitor to stop the reaction, aliquots were injected to C₁₈ column and the amounts of the intact diastereomers were evaluated using their absorbance at 215 nm. The results summarized in **Table 7** show that the diastereomers of the invention are significantly less susceptible to proteases digestion as compared to the native antimicrobial peptide cecropin B.

**Table 7**

| **Proteolytic digestion (%) of the diastereomers** | | | |
|---|---|---|---|
| **Peptide designation** | **Trypsin** | **Pepsin** | **Elastase** |
| **2** | 32 | 10 | 6 |
| **Cecropin B** | 100 | 100 | 100 |

### Example 6. Activity of the diastereomers of the invention against Mycoplasma

Mycoplasmas are the smallest free-living microorganisms, being about 300 nm in diameter. They are bounded by a triple-layered membrane and, unlike conventional bacteria, do not have a rigid cell wall. Hence, they are not susceptible to penicillins and other antibiotics that act on bacteria. Mycoplasma infection of cell cultures is widespread and has major detrimental effects on cellular physiology and metabolism. Since cell culture is used extensively, both in research and in industrial production processes, it is essential to find a way to eliminate mycoplasma contamination.

In order to examine the activity of the diastereomers of the invention against mycoplasma, 500 µl of *mycoplasma pneumoniae* cells were introduced into a cell culture flask containing 50 ml of SP-4 growth media (Jacob et al., 1985). The cells were grown at 37°C for 3-4 days. Before the assay, the mycoplasma cells were washed 3 times with serum-free medium (without fetal calf serum) and then harvested into 5 ml depleted-medium. The cells' concentration was adjusted to approximately 1x10⁶ cell ml ⁻¹. Thereafter, 150 µl of either diastereomer **1** or **2** in phosphate-buffered saline (PBS) were added to 150 µl mycoplasma cells. The mixture was incubated for 1 hour at room temperature. As a control, cells were incubated with PBS alone. At the end of the incubation period, 150 µl of the mixture was inoculated into tissue culture flasks (in duplicate) containing 15 ml SP-4 media. After 3-4 days, the mycoplasma viability was determined by counting the number of cells grown in each flask. Anti-mycoplasma activity is expressed as the minimal inhibitory concentration (MIC), the concentration at which 100 % inhibition of growth was observed after the incubation time mentioned above.

As shown in **Table 8**, the 12-mer diastereomer **1** known from the prior art was less active against mycoplasama than the 15-mer peptide **2** of the present invention. Moreover, compared to their activity against normal cell line **(Table 3),** both peptides were active against NIH-3T3 fibroblasts at concentrations that are 2-fold higher than the concentration at which they act against mycoplasma, indicating that the diastereomeric peptides have a selective activity against mycoplasma.

**Table 8**

| **MIC (µg/ml) of peptides 1 and 2 against Mycoplasma.** | |
|---|---|
| **Peptide designation** | **Mycoplasma** |
| **1** | 50 |
| **2** | 12.5 |

### Example 7. Effect of diastereomers of the invention on primary skin and eye irritation in rabbits

The purpose of this experiment was to provide information on the irritation likely to arise from a single instillation of peptide **2** into the eye or from a single topical exposure to it. The study was conducted by the service company "PSL" (Product Safety Labs, 725 Cranbury Road, East Brunswick, New Jersey, USA).

For the eye irritation assay, three healthy rabbits (1 male and 2 female) without pre-existing ocular irritation were selected. Prior to instillation, peptide **2** was dissolved in 0.9% saline to result in a 0.5% concentration, then mixed well using a vortex mixer. One-tenth of a milliliter of the prepared test substance was instilled into the conjunctival sac of the right eye of each rabbit by pulling the lower lid away from the eyeball. The upper and lower lids were then gently held together for about one second before releasing, to minimize loss of the test substance. The left eye remained untreated and served as a control. Ocular irritation was evaluated with the illumination of a white light source at 1, 24, 48 and 72 hours after the instillation according to the "Scale for scoring ocular lesions" used in "PSL". Fluorescein dye was used at 24 hours to verify the absence of corneal damage.

For the skin irritation assay, three healthy rabbits were selected. On the day before application, the fur on the treatment site of each animal was removed by clipping. Care was taken to avoid abrading the skin. After clipping and prior to application, the animal's skin was checked for any abnormalities according to the "Primary Skin Irritation Scoring System" used in "PSL". If any site scores were greater than zero, that animal was removed from test and replaced. Prior to the exposure, peptide 2 was dissolved in 0.9% saline to result in a 0.5% concentration, then mixed well using a vortex mixer. Five-tenths of a milliliter of the prepared test substance was applied directly to the skin of the treatment site. The entire trunk of each animal was then wrapped with non-irritating occlusive adhesive tape to avoid dislocation of the patch. Animals were exposed to the test substance for a period of 24 hours. Elizabethan collars were placed on each rabbit for the designated exposure period. Following the exposure period, the patches were removed and the treatment sites were wiped with water or appropriate solvents using clean towels to remove any residual test substance. The treatment sites were examined for signs of erythema and edema within 1 hour and at approximately 24, 48 and 72 hours after patch removal according to the "Primary Skin Irritation Scoring System" developed by Draize (Draize et al., 1944). Individual scores were recorded for each rabbit. A narrative description of pertinent skin observations and any signs of gross toxicity were recorded. In addition, evaluation of the reversibility or irreversibility of the observed effects was noted.

According to the results obtained, no corneal opacity or iritis was noted during the eye irritation study and no signs of erythema and edema were observed during the skin irritation assay. Thus, it can be concluded that peptide **2** does not cause eye or skin irritation in the high concentration of 0.5% (5 mg/ml).

### Example 8. Liposome encapsulation of the diastereomeric peptides

Liposomes serve as convenient delivery vehicles for biologically active molecules. Hydrophilic drugs can be encapsulated in the internal aqueous compartment, whereas hydrophobic drugs may bind to or are incorporated in the lipid bilayers. In this experiment, liposomal diastereomeric peptides were prepared in order to lower the peptide toxicity and increase their selectivity.

Liposomes composed of different ratios of phosphatidylcholine (PC)/phosphatidylglycerol (PG) (9:1; 4:1; 1:1 w/w) or phosphatidylethanolamine (PE)/PG (9:1; 4:1; 1:1 w/w) were prepared. In brief, dry lipid mixtures were dissolved in CHCl₃/MeOH (2/1, v/v). The solvents were evaporated under nitrogen stream, and the lipid mixtures at the compositions described above were resuspended in PBS by vortex mixing. The lipid suspension was extruded through 3 different polycarbonate filters (1 µm, 0.2 µm and 0.1 µm pore size filters, 15 times each). Finally, the resulting suspensions of large unilamellar vesicles (LUV) were added to different concentrations of peptide **9** to give lipid/peptide ratios of 50:1; 30:1; 10:1 w/w, respectively. The mixtures were sonicated for 2 minutes and the liposomes were stored at **4⁰C** until used.

The antibacterial activity of the resulting liposomal diastereomeric peptide preparations were examined as described in Example 2 above. The MICs of liposomal peptide **9** in various lipid compositions and peptide/lipid ratios (as described above), or of liposomes at lipid compositions equivalent to the loaded liposomes (not shown) or peptide **9** alone, were determined using the following bacteria: *Acinetobacter baumannii* ATCC 19606, and *Staphylocuccus aureus* ATCC 6538P.

The results summarized in Table 9 show that liposomal peptide **9** composed of the lipids PC/PG (9:1 w/w) at a 10:1 w/w lipid/peptide ratio, exhibits MIC results similar to those of peptide **9** alone. Hence, peptide **9** entrapped within liposomes can maintain its antibacterial activity, however this activity is contingent on the liposomes' lipid composition and on the lipid/peptide ratio.

**Table 9**

| **MIC (µg/ml) of liposomal peptide 9 for bacteria** | | | |
|---|---|---|---|
| **Liposome Composition (W/W)** | **Lipid/peptide Ratio (W/W)** | **A. baumannii** | **S. aureus** |
| PC/PG 4:1 | 30:1 | 6.25 | 50 |
| PC/PG 9:1 | 50:1 | 3.1 | 50 |
| PC/PG 4:1 | 50:1 | 12.5 | 50 |
| **PC/PG 9:1** | **10:1** | **3.1** | **12.5** |
| | **Peptide 9 alone** | **3.1** | **12.5** |

The *in vivo* toxicity of the liposomal peptide **9** preparation composed of the lipids PC/PG (9:1 w/w) at a 10:1 w/w lipid/peptide ratio was examined. Groups of 5 CD1 male mice weighing 24-27 g (5-week old), bred in an animal isolator (IVC racks) under specific pathogen-free (SPF) conditions at 24 ± 1⁰C, were used. Twelve mg/kg liposomal peptide **9** or peptide **9** alone dissolved in PBS in a dosing volume of 10 ml/kg, were administered by single i.v. bolus injection via the mice tail vein. In parallel, control groups received i.v. injections of equivalent liposomes alone or PBS in a dosing volume of 10 ml/kg.

The results in Table 10 show that 12 mg/kg of peptide **9** injected i.v. caused 80% mortality, while liposomal peptide **9** in the same concentration produced only 20% mortality. No incidence of mortality occurred following the i.v. injection of PBS or the liposomes alone. These results show that entrapment of diastereomeric peptides of the invention in liposomes can reduce their toxicity while maintaining their activity.

**Table 10**

| **Incidence of mortality in mice following single i.v. injection of liposomal peptide 9, peptide 9, liposomes, or PBS** | |
|---|---|
| **Treatment** | **Incidence of mortality*** |
| **Liposomal peptide 9 (12 mg/kg)** | 1/5 |
| **Peptide 9 (12 mg/kg)** | 4/5 |
| **Liposomes** | 0/5 |
| PBS | 0/5 |

| | |
|---|---|
| * No. animals affected / No. animals per group. | |

### References

Draize J. H., Woodward, G. & Calvery, H. O. (1944). *J Pharmacol Exp Ther* **82**, 377-390.
Hong, J., Oren, Z. & Shai, Y. (1999). *Biochemistry.*
Jacob, E., Schopperele, K. & Bredt, W. (1985). *Eur. J. Clin. Microbiol.* **4,** 113-118. Oren, Z. & Shai, Y. (1996). *Biochemistry* **36,** 1826-1835.
Oren, Z. & Shai, Y. (2000). *Biochemistry* **39**, 6103-6114.
Oren, Z., Hong, J. & Shai, Y. (1997). *J*. *Biol. Chem.* **272,** 14643-14649.
Porgador, A., Bannerj, R., Watanable, Y., Feldman, M., Gilboa, E. & Eisenbach, L
(1993) *J*. Immunol. **150,** 1458-1470
Raff, M. C. (1971). *Transpl. Rev.* **6,** 52-80.
Shai, Y. & Oren, Z. (1996). *J*. *Biol. Chem.* **271,** 7305-7308.
Sharon, M., Oren, Z., Shai, Y. & Anglister, J. (1999). *Biochemistry* **38,** 15303-15316.
Utsugi, T., Schroit, A. J., Connor, J., Bucana, C. D. & Fidler, I. J. (1991). *Cancer Res.* **51,** 3062-3066.

## Claims

1. A diastereomeric peptide with a net positive charge greater than +1 and having at least 15 amino acid residues, wherein said residues are selected from: (i) leucine and lysine; (ii) leucine, lysine and arginine ; (iii) the residues of (i) or (ii) and glycine, serine, aspargine, threonine, or glutamine at the N-terminus; or (iv) the residues of (i), (ii) or (iii) and lysine, arginine, glycine, or serine at the C-terminus; and (v) cyclic analogues of (i), (ii), (iii) or (iv).

2. A diastereomeric peptide according to claim 1 composed of leucine and lysine residues.

3. A diastereomeric peptide according to claim 2, selected from the peptides of the sequences:
Lys-Leu-**Leu**-Leu-Lys-Leu-Leu-**Lys-Lys**-Leu-Leu-Lys-**Leu**-Leu-Lys-NH_{2 **2**}
Leu-Lys-**Leu**-Leu-Lys-Lys-**Leu-Leu**-Lys-Lys-Leu-Leu-**Lys**-Leu-Leu-NH_{2 **3**}
Leu-Lys-**Leu**-Leu-Lys-**Lys**-Leu-**Leu-Lys**-Lys-Leu-**Leu**-Lys-Leu-Leu-NH_{2 **4**}
Lys-Leu-**Leu**-Lys-Leu-Leu-**Lys-Lys**-Leu-**Leu**-Lys-Leu-**Leu**-Leu-Lys-NH_{2 **7**}
Lys-Leu-**Leu**-Leu-Lys-**L*e*u**-Leu-**Lys-Lys**-Leu-Leu-Lys-**Leu**-Leu-Lys-NH_{2 **10**}

4. A diastereomeric peptide according to claim 1 composed of leucine, lysine and arginine residues.

5. A diastereomeric peptide according to claim 4, selected from the peptides of the sequences:
Lys-Leu-**Leu**-Leu-Arg-**Leu**-Leu-**Lys-Lys**-Leu-Leu-Arg-**Leu**-Leu-Lys-NH_{2 **6**}
Lys-Leu-**Leu**-Arg-Leu-Leu-**Lys-Lys**-Leu-**Leu**-Arg-Leu-**Leu**-Leu-Lys-NH_{2 **9**}

6. A diastereomeric peptide according to claim 1 composed of leucine and lysine or of leucine, lysine and arginine residues, and having at the N-terminus an amino acid selected from glycine, serine, aspargine, threonine, or glutamine.

7. A diastereomeric peptide according to claim 6 wherein said amino acid at the N-terminus is glycine.

8. A diastereomeric peptide according to claim 7, selected from the peptides of the sequences:
Gly-Leu-**Leu**-Leu-Arg-**Leu**-Leu-**Lys**-**Lys**-Leu-Leu-Arg-**Leu**-Leu-Lys-Lys-NH_{2 **5**}
Gly-Leu-**Leu**-Lys-Leu-Leu-**Lys-Lys**-Leu-**Leu**-Lys-Leu-**Leu**-Leu-Lys-Lys-NH_{2 **8**}

9. A diastereomeric peptide according to claim 1 wherein the peptide is a cyclic analogue of said diastereomeric peptide of (i), (ii), (iii) or (iv).

10. A diastereomeric peptide according to claim 9, selected from the peptides of the sequences:

Cys-Lys-Leu-Leu- Leu-Lys-Leu-Leu-Lys-Lys- Leu-Leu-Lys-Leu- Leu-Lys-Cys

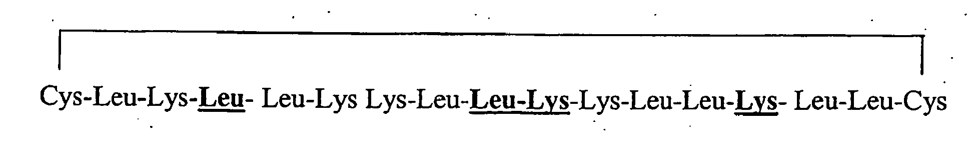
Cys-Leu-Lys-Leu- Leu-Lys Lys-Leu-Leu-Lys-Lys-Leu-Leu-Lys- Leu-Leu-Cys

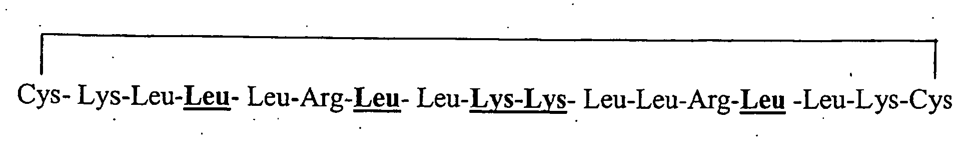
Cys- Lys-Leu-Leu- Leu-Arg-Leu- Leu-Lys-Lys- Leu-Leu-Arg-Leu -Leu-Lys-Cys

11. A pharmaceutical composition comprising a diastereomeric peptide according to any- one of claims 1-to 10, and a pharmaceutically acceptable carrier.

12. A pharmaceutical composition according to claim 11 for topical application.

13. A pharmaceutical composition according to claim 12 for topical treatment of: acne; topical infections caused by pathogenic organisms such as bacterial infections including chronic gastric mucosal infestation by *Helicobacter pylori,* intestinal bacterial infections, infections caused by antibiotic-resistant bacteria e.g. *Streptococcus pyogenes* and the methicilin-resistant *Staphylococcus aureus;* fungal infections including nail fungi, infections caused by yeasts such as *Candida albicans,* fungal infections of the scalp; fungal or bacterial infections related to surgical or traumatic wounds; chronic or poorly healing skin lesions such as foot ulcer in diabetes mellitus patients; vaginal infection (vaginitis); eye and ear infections; burn wounds; infections of mouth and throat; and localized infections such as chronic pulmonary infections in cystic fibrosis, emphysema and asthma.

14. A pharmaceutical composition according to claim 13 for treatment of bacterial infections comprising a diastereomeric peptide selected from the peptides of the sequences:
Lys-Leu-**Leu**-Leu-Lys-Leu-Leu-**Lys-Lys**-Leu-Leu-Lys-**Leu**-Leu-Lys-NH_{2 **2**}
Leu-Lys-**Leu**-Leu-Lys-Lys-Leu-**Leu-Lys**-Lys-Leu-Leu-**Lys**-Leu-Leu-NH_{2 **3**}
Leu-Lys-**Leu**-Leu-Lys-**Lys**-Leu-**Leu-Lys**-Lys-Leu-Leu-**Lys**-Leu-Leu-NH_{2 **4**}
Lys-Leu-**Leu-**Leu-Arg-**Leu**-Leu-**Lys-Lys**-Leu-Leu-Arg-**Leu**-Leu-Lys-NH_{2 **6**}
Lys-Leu-**Leu-**Lys-Leu-Leu-**Lys-Lys**-Leu-**Leu-**Lys-Leu-**Leu**-Leu-Lys-NH_{2 **7**}
Gly-Leu-**Leu-**Leu-Arg-**Leu**-Leu-**Lys-Lys**-Leu-Leu-Arg-**Leu**-Leu-Lys-Lys-NH_{2 **5**}
Gly-Leu-**Leu**-Lys-Leu-Leu-**Lys-Lys**-Leu-**Leu**-Lys-Leu-**Leu**-Leu-Lys-Lys-NH_{2 **8**}

15. A pharmaceutical composition according to claim 13 or 14 for treatment of infections caused by antibiotic-resistant bacteria comprising a diastereomeric peptide selected from the peptides of the sequences:
Lys-Leu-**Leu**-Leu-Lys-Leu-Leu-**Lys-Lys**-Leu-Leu-Lys-**Leu**-Leu-Lys-NH_{2 **2**}
Lys-Leu-**Leu**-Leu-Arg-**Leu**-Leu-**Lys-Lys**-Leu-Leu-Arg-**Leu**-Leu-Lys-NH_{2 **6**}

16. A pharmaceutical composition according to claim 13 for treatment of fungal infections comprising a diastereomeric peptide selected from the peptides of the sequences:
Lys-Leu-**Leu**-Leu-Lys-Leu-Leu-**Lys-Lys**-Leu-Leu-Lys-**Leu**-Leu-Lys-NH_{2 **2**}
Leu-Lys-**Leu**-Leu-Lys-Lys-Leu-**Leu-Lys**-Lys-Leu-Leu-**Lys**-Leu-Leu-NH_{2 **3**}
Leu-Lys-**Leu**-Leu-Lys-**Lys**-Leu-**Leu-Lys**-Lys-Leu-Leu-**Lys**-Leu-Leu-NH_{2 **4**}
Lys-Leu-**Leu**-Leu-Arg-**Leu**-Leu-**Lys-Lys**-Leu-Leu-Arg-**Leu**-Leu-Lys-NH_{2 **6**}
Lys-Leu-**Leu**-Lys-Leu-Leu-Leu-**Lys-Lys**-Leu-**Leu**-Lys-Leu-**Leu**-Leu-Lys-NH_{2 **7**}
Gly-Leu-**Leu-**Leu-Arg-**Leu**-Leu-**Lys-Lys**-Leu-Leu-Arg-**Leu**-Leu-Lys-Lys-NH_{2 **5**}
Gly-Leu-**Leu**-Lys-Leu-Leu-**Lys-Lys**-Leu-**Leu**-Lys-Leu-**Leu**-Leu-Lys-Lys-NH_{2 **8**}

17. A pharmaceutical composition according to any one of the claims 11 to 16 in the form of solution; colloidal dispersion, cream, lotion, gel, foam, mousse, spray, aerosol or other formulation for nasal or pulmonary application.

18. A pharmaceutical composition according to claim 11 for the treatment of cancer.

19. An anticancer pharmaceutical composition according to claim 18 comprising a diastereomeric peptide selected from the peptides of the sequences:
Lys-Leu-**Leu**-Leu-Lys-Leu-Leu-**Lys-Lys**-Leu-Leu-Lys-**Leu**-Leu-Lys-NH_{2 **2**}
Gly-Leu-**Leu**-Lys-Leu-Leu-**Lys-Lys**-Leu-**Leu**-Lys-Leu-**Leu**-Leu-Lys-Lys-NH_{2 **8**}
Lys-Leu-**Leu**-Arg-Leu-Leu-**Lys-Lys**-Leu-**Leu**-Arg-Leu-**Leu**-Leu-Lys-NH_{2 **9**}
Lys-Leu-**Leu**-Leu-Lys-**Leu**-Leu-**Lys-Lys**-Leu-Leu-Lys-**Leu**-Leu-Lys-NH_{2 **10**}

20. A pharmaceutical composition according to claim 18 or 19 for the treatment of melanoma, basal and squamous cell carcinomas, or for treatment of breast, colon, lung, or prostate tumors.

21. A pharmaceutical composition according to claim 20 for treatment of breast, colon, lung melanoma or prostate tumors, comprising a diastereomeric peptide selected from the peptides of the sequences:
Lys-Leu-**Leu**-Arg-Leu-Leu-**Lys-Lys**-Leu-Leu-Arg-Leu-**Leu**-Leu-Lys-NH_{2 **9**}
Lys-Leu-**Leu**-Leu-Lys-**Leu**-Leu-**Lys-Lys**-Leu-Leu-Lys-**Leu**-Leu-Lys-NH_{2 **10**}

22. A composition comprising a diastereomeric peptide according to any one of claims 1 to 10 to control mycoplasma infection in cell culture, for food preservation, or for use as food supplement.

23. A veterinary composition comprising a diastereomeric peptide according to any one of claims 1 to 10.

## Patentansprüche

1. Diastereomeres Peptid mit einer positiven Gesamtladung von größer als +1 und mit mindestens 15 Aminosäureresten, wobei die Reste ausgewählt sind aus: (i) Leucin und Lysin; (ii) Leucin, Lysin und Arginin; (iii) den Resten aus (i) oder (ii) und Glycin, Serin, Asparagin, Threonin oder Glutamin am N-Terminus; oder (iv) den Resten aus (i), (ii) oder (iii) und Lysin, Arginin, Glycin oder Serin am C-Terminus; und (v) cyclischen Analoga von (i), (ii), (iii) oder (iv).

2. Diastereomeres Peptid gemäß Anspruch 1, bestehend aus Leucin- und Lysinresten.

3. Diastereomeres Peptid gemäß Anspruch 2, ausgewählt aus den Peptiden der Sequenzen:
Lys-Leu-**Leu**-Leu-Lys-Leu-Leu-**Lys-Lys**-Leu-Leu-Lys-**Leu**-Leu-Lys-NH_{2 **2**}
Leu-Lys-**Leu**-Leu-Lys-Lys-Leu-**Leu-Lys**-Lys-Leu-Leu-**Lys**-Leu-Leu-NH_{2 **3**}
Leu-Lys-**Leu**-Leu-Lys-**Lys**-Leu-**Leu-Lys**-Lys-Leu-Leu-**Lys**-Leu-Leu-NH_{2 **4**}
Lys-Leu-**Leu**-Lys-Leu-Leu-**Lys-Lys**-Leu-**Leu**-Lys-Leu-**Leu**-Leu-Lys-NH_{2 **7**}
Lys-Leu-**Leu**-Leu-Lys-**Leu**-Leu-**Lys-Lys**-Leu-Leu-Lys-**Leu**-Leu-Lys-NH_{2 **10**}

4. Diastereomeres Peptid gemäß Anspruch 1, bestehend aus Leucin-, Lysin- und Argininresten.

5. Diastereomeres Peptid gemäß Anspruch 4, ausgewählt aus den Peptiden der Sequenzen:
Lys-Leu-**Leu**-Leu-Arg-**Leu**-Leu-**Lys-Lys**-Leu-Leu-Arg-**Leu**-Leu-Lys-NH_{2 **6**}
Lys-Leu-**Leu**-Arg-Leu-Leu-**Lys-Lys**-Leu-**Leu**-Arg-Leu-**Leu**-Leu-Lys-NH_{2 **9**}

6. Diastereomeres Peptid gemäß Anspruch 1, bestehend aus Leucin- und Lysin- oder aus Leucin-, Lysin- und Argininresten und mit einer Aminosäure, ausgewählt aus Glycin, Serin, Asparagin, Threonin oder Glutamin, am N-Terminus.

7. Diastereomeres Peptid gemäß Anspruch 6, wobei die Aminosäure am N-Terminus Glycin ist.

8. Diastereomeres Peptid gemäß Anspruch 7, ausgewählt aus den Peptiden der Sequenzen:
Gly-Leu-**Leu**-Leu-Arg-**Leu**-Leu-**Lys-Lys**-Leu-Leu-Arg-**Leu**-Leu-Lys-Lys-NH_{2 **5**}
Gly-Leu-**Leu**-Lys- Leu-Leu-**Lys-Lys**-Leu-**Leu**-Lys-Leu-**Leu**-Leu-Lys-Lys-NH_{2 **8**}

9. Diastereomeres Peptid gemäß Anspruch 1, wobei das Peptid ein cyclisches Analogon des diastereomeren Peptids aus (i), (ii), (iii) oder (iv) ist.

10. Diastereomeres Peptid gemäß Anspruch 9, ausgewählt aus den Peptiden der Sequenzen:

Cys-Lys-Leu-Leu- Leu-Lys-Leu-Leu-Lys-Lys- Leu-Leu-Lys-Leu- Leu-Lys-Cys

Cys-Leu-Lys-Leu- Leu-Lys Lys-Leu-Leu-Lys-Lys-Leu-Leu-Lys- Leu-Leu-Cys

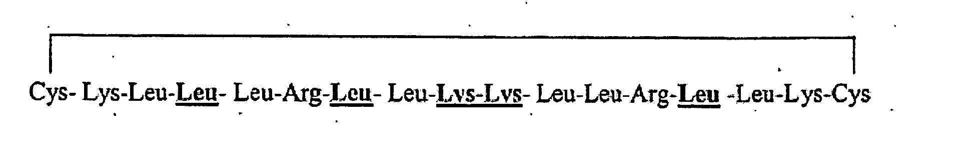

Cys- Lys-Leu-Leu- Leu-Arg-Leu- Leu-Lys-Lys- Leu-Leu-Arg-Leu -Leu-Lys-Cys

11. Arzneimittel, umfassend ein diastereomeres Peptid gemäß einem der Ansprüche 1 bis 10, und einen pharmazeutisch verträglichen Träger.

12. Arzneimittel gemäß Anspruch 11 zur topischen Verabreichung.

13. Arzneimittel gemäß Anspruch 12 zur topischen Behandlung von Akne; topischen Infektionen, die durch pathogene Organismen verursacht werden, wie bakterielle Infektionen, einschließlich chronischem Befall der Magenschleimhaut durch *Helicobacter pylori,* bakteriellen Darminfektionen, Infektionen, die durch gegen Antibiotika resistente Bakterien, z.B. *Streptococcus pyogenes,* und die durch gegen Methicilin resistente *Staphylococcus aureus* verursacht werden; Pilzinfektionen einschließlich Nagelpilze; durch Hefen, wie *Candida albicans,* verursachten Infektionen; Pilzinfektionen der Kopfhaut; mit chirurgischen oder traumatischen Wunden in Verbindung stehenden Pilz- oder bakteriellen Infektionen; chronischen oder schlecht heilenden Hautverletzungen wie Fußgeschwür bei Patienten mit Diabetes mellitus; vaginalen Infektionen (Vaginitis); Augen- und Ohrinfektionen; Brandwunden; Infektionen des Mundes und Halses; und lokalisierten Infektionen wie chronischen Lungeninfektionen bei Mukoviszidose, Emphysemen und Asthma.

14. Arzneimittel gemäß Anspruch 13 zur Behandlung von bakteriellen Infektionen, umfassend ein diastereomeres Peptid, ausgewählt aus den Peptiden der Sequenzen:
Lys-Leu-**Leu**-Leu-Lys-Leu-Leu-**Lys-Lys**-Leu-Leu-Lys-**Leu**-Leu-Lys-NH_{2 **2**}
Leu-Lys-**Leu**-Leu-Lys-Lys-Leu-**Leu-Lys**-Lys-Leu-Leu-**Lys**-Leu-Leu-NH_{2 **3**}
Leu-Lys-**Leu**-Leu-Lys-**Lys**-Leu-**Leu-Lys**-Lys-Leu-Leu-**Lys**-Leu-Leu-NH_{2 **4**}
Lys-Leu-**Leu**-Leu-Arg-**Leu**-Leu-**Lys-Lys**-Leu-Leu-Arg-**Leu**-Leu-Lys-NH_{2 **6**}
Lys-Leu-**Leu**-Lys-Leu-Leu-**Lys-Lys**-Leu-**Leu**-Lys-Leu-**Leu**-Leu-Lys-NH_{2 **7**}
Gly-Leu-**Leu**-Leu-Arg-**Leu**-Leu-**Lys-Lys**-Leu-Leu-Arg-**Leu**-Leu-Lys-Lys-NH_{2 **5**}
Gly-Leu-**Leu**-Lys-Leu-Leu-**Lys-Lys**-Leu-**Leu**-Lys-Leu-**Leu**-Leu-Lys-Lys-NH_{2 **8**}

15. Arzneimittel gemäß Anspruch 13 oder 14 zur Behandlung von durch gegen Antibiotika resistente Bakterien verursachten Infektionen, umfassend ein diastereomeres Peptid, ausgewählt aus den Peptiden der Sequenzen:
Lys-Leu-**Leu-**Leu-Lys-Leu-Leu-**Lys-Lys**-Leu-Leu-Lys-**Leu**-Leu-Lys-NH_{2 **2**}
Lys-Leu-**Leu**-Leu-Arg-**Leu**-Leu-**Lys-Lys**-Leu-Leu-Arg-**Leu**-Leu-Lys-NH_{2 **6**}

16. Arzneimittel gemäß Anspruch 13 zur Behandlung von Pilzinfektionen, umfassend ein diastereomeres Peptid, ausgewählt aus den Peptiden der Sequenzen:
Lys-Leu-**Leu**-Leu-Lys-Leu-Leu-**Lys-Lys**-Leu-Leu-Lys-**Leu**-Leu-Lys-NH_{2 **2**}
Leu-Lys-**Leu**-Leu-Lys-Lys-Leu-**Leu-Lys**-Lys-Leu-Leu-**Lys**-Leu-Leu-NH_{2 **3**}
Leu-Lys-**Leu**-Leu-Lys-**Lys**-Leu-**Leu-Lys**-Lys-Leu-Leu-**Lys**-Leu-Leu-NH_{2 **4**}
Lys-Leu-**Leu**-Leu-Arg-**Leu**-Leu-**Lys-Lys**-Leu-Leu-Arg-**Leu**-Leu-Lys-NH_{2 **6**}
Lys-Leu-**Leu**-Lys-Leu-Leu-**Lys-Lys**-Leu-**Leu**-Lys-Leu-**Leu**-Leu-Lys-NH_{2 **7**}
Gly-Leu-**Leu**-Leu-Arg-**Leu**-Leu-**Lys-Lys**-Leu-Leu-Arg-**Leu**-Leu-Lys-Lys-NH_{2 **5**}
Gly-Leu-**Leu**-Lys-Leu-Leu-**Lys-Lys**-Leu-**Leu**-Lys-Leu-**Leu**-Leu-Lys-Lys-NH_{2 **8**}

17. Arzneimittel gemäß einem der Ansprüche 11 bis 16 in Form einer Lösung, einer kolloidalen Dispersion, Creme, Lotion, eines Gels, Schaums, Mousse, Sprays, Aerosols oder anderen Formulierung für die nasale Anwendung oder die Anwendung in der Lunge.

18. Arzneimittel gemäß Anspruch 11 zur Behandlung von Krebs.

19. Arzneimittel gegen Krebs gemäß Anspruch 18, umfassend ein diastereomeres Peptid, ausgewählt aus den Peptiden der Sequenzen:
Lys-Leu-**Leu**-Leu-Lys-Leu-Leu-**Lys-Lys**-Leu-Leu-Lys-**Leu**-Leu-Lys-NH_{2 **2**}
Gly-Leu-**Leu**-Lys-Leu-Leu-**Lys-Lys**-Leu-**Leu**-Lys-Leu-Leu-**Leu**-Lys-Lys-NH_{2 **8**}
Lys-Leu-**Leu**-Arg-Leu-Leu-**Lys-Lys**-Leu-**Leu**-Arg-Leu-**Leu**-Leu-Lys-NH_{2 **9**}
Lys-Leu-**Leu**-Leu-Lys-**Leu**-Leu-**Lys-Lys**-Leu-Leu-Lys-**Leu**-Leu-Lys-NH_{2 **10**}

20. Arzneimittel gemäß Anspruch 18 oder 19 zur Behandlung von Melanom-, Basal- oder Plattenepithelzellenkarzinomen oder zur Behandlung von Brust-, Darm-, Lungen- oder Prostatatumoren.

21. Arzneimittel gemäß Anspruch 20 zur Behandlung von Brust-, Darm-, Lungenmelanomen oder Prostatatumoren, umfassend ein diastereomeres Peptid, ausgewählt aus den Peptiden der Sequenzen:
Lys-Leu-**Leu**-Arg-Leu-Leu-**Lys-Lys**-Leu-Leu-Arg-Leu-**Leu**-Leu-Lys-NH_{2 **9**}
Lys-Leu-**Leu**-Leu-Lys-**Leu**-Leu-**Lys-Lys**-Leu-Leu-Lys-**Leu**-Leu-Lys-NH_{2 **10**}

22. Zusammensetzung, umfassend ein diastereomeres Peptid gemäß einem der Ansprüche 1 bis 10 zur Steuerung einer Mycoplasmainfektion in einer Zellkultur, zur Nahrungsmittelkonservierung oder zur Verwendung als Nahrungsergänzungsmittel.

23. Veterinärmedizinische Zusammensetzung, umfassend ein diastereomeres Peptid gemäß einem der Ansprüche 1 bis 10.

## Revendications

1. Peptide diastéréomère avec une charge positive nette supérieure à +1 et ayant au moins 15 résidus d'acides aminés, dans lequel lesdits résidus sont choisis parmi : (i) la leucine et la lysine ; (ii) la leucine, la lysine et l'arginine, (iii) les résidus de (i) ou (ii) et la glycine, la sérine, l'asparagine, la thréonine ou la glutamine au niveau du N-terminus ; ou (iv) les résidus de (i), (ii) ou (iii) et la lysine, l'arginine, la glycine ou la sérine au niveau du C-terminus ; et (v) les analogues cycliques de (i), (ii), (iii) ou (iv).

2. Peptide diastéréomère selon la revendication 1, constitué de résidus leucine et lysine.

3. Peptide diastéréomère selon la revendication 2, sélectionné parmi les peptides de séquences :
Lys-Leu-**Leu**-Leu-Lys-Leu-Leu-**Lys-Lys**-Leu-Leu-Lys-**Leu**-Leu-Lys-NH_{2 **2**}
Leu-Lys-**Leu**-Leu-Lys-Lys-Leu-**Leu-Lys**-Lys-Leu-Leu-**Lys**-Leu-Leu-NH_{2 **3**}
Leu-Lys-**Leu**-Leu-Lys-**Lys**-Leu-**Leu-Lys**-Lys-Leu-Leu-**Lys**-Leu-Leu-NH_{2 **4**}
Lys-Leu-**Leu**-Lys-Leu-Leu-**Lys-Lys**-Leu-**Leu**-Lys-Leu-**Leu**-Leu-Lys-NH_{2 **7**}
Lys-Leu-**Leu-**Leu-Lys-**Leu**-Leu-**Lys-Lys**-Leu-Leu-Lys-**Leu**-Leu-Lys-NH_{2 **10**}

4. Peptide diastéréomère selon la revendication 1, constitué de résidus leucine, lysine et arginine.

5. Peptide diastéréomère selon la revendication 4, sélectionné parmi les peptides de séquences :
Lys-Leu-**Leu**-Leu-Arg-**Leu**-Leu-**Lys-Lys**-Leu-Leu-Arg-**Leu**-Leu-Lys-NH_{2 **6**}
Lys-Leu-**Leu**-Arg-Leu-Leu-**Lys-Lys**-Leu-**Leu**-Arg-Leu-**Leu**-Leu-Lys-NH_{2 **9**}

6. Peptide diastéréomère selon la revendication 1, constitué de leucine et de lysine ou de résidus leucine, lysine et arginine et ayant au niveau du N-terminus un acide aminé choisi parmi la glycine, la sérine, l'asparagine, la thréonine ou la glutamine.

7. Peptide diastéréomère selon la revendication 6, dans lequel ledit acide aminé au niveau du N-terminus est la glycine.

8. Peptide diastéréomère selon la revendication 7, choisi parmi les peptides de séquences :
Gly-Leu-**Leu**-Leu-Arg-**Leu**-Leu-**Lys-Lys**-Leu-Leu-Arg-**Leu**-Leu-Lys-Lys-NH_{2 **5**}
Gly-Leu-**Leu**-Lys-Leu-Leu-**Lys-Lys**-Leu-**Leu**-Lys-Leu-**Leu**-Leu-Lys-Lys-NH_{2 **8**}

9. Peptide diastéréomère selon la revendication 1, dans lequel le peptide est un analogue cyclique dudit peptide diastéréomère de (i), (ii), (iii) ou (iv).

10. Peptide diastéréomère selon la revendication 9, sélectionné parmi les peptides de séquences :

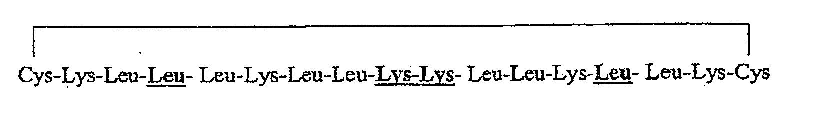

Cys-Lys-Leu-Leu- Leu-Lys-Leu-Leu-Lys-Lys- Leu-Leu-Lys-Leu- Leu-Lys-Cys

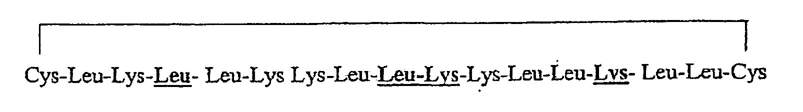
Cys-Leu-Lys-Leu- Leu-Lys Lys-Leu-Leu-Lys-Lys-Leu-Leu-Lvs- Leu-Leu-Cys

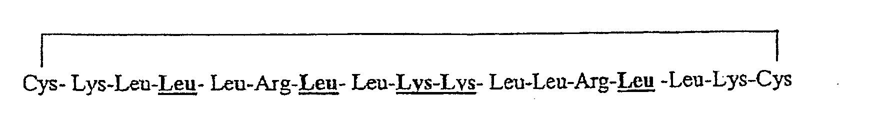
Cys- Lys-Leu-Leu- Leu-Arg-Leu- Leu-Lys-Lys- Leu-Leu-Arg-Leu -Leu-Lys-Cys

11. Composition pharmaceutique comprenant un peptide diastéréomère selon l'une quelconque des revendications 1 à 10, et un support pharmaceutiquement acceptable.

12. Composition pharmaceutique selon la revendication 11, pour une application topique.

13. Composition pharmaceutique selon la revendication 12, pour le traitement topique: de l'acné ; des infections topiques provoquées par des organismes pathogènes telles que des infections bactériennes comprenant l'infestation chronique des muqueuses gastriques par *Helicobacter pylori,* les infections bactériennes intestinales, les infections provoquées par des bactéries résistantes aux antibiotiques, par exemple *Streptococcus pyogenes* et la bactérie résistante à la méthiciline *Staphylococcus aureus*; des infections fongiques comprenant les mycoses des ongles, les infections provoquées par des levures telles que *Candida albicans,* les infections fongiques du cuir chevelu ; des infections fongiques ou bactériennes liées aux blessures chirurgicales ou traumatiques ; des lésions cutanées chroniques ou lentes à cicatriser telles que l'ulcère du pied chez les patients souffrant de diabète sucré ; de l'infection vaginale (vaginite) ; des infections oculaires et auriculaires ; des brûlures ; des infections de la bouche et de la gorge ; et des infections localisées telles que les infections pulmonaires chroniques dans le cas de mucoviscidose, d'emphysème et d'asthme.

14. Composition pharmaceutique selon la revendication 13, pour le traitement des infections bactériennes comprenant un peptide diastéréomère choisi parmi les peptides de séquences :
Lys-Leu-**Leu**-Leu-Lys-Leu-Leu-**Lys-Lys**-Leu-Leu-Lys-**Leu**-Leu-Lys-NH_{2 **2**}
Leu-Lys-**Leu**-Leu-Lys-Lys-Leu-**Leu-Lys**-Lys-Leu-Leu-**Lys**-Leu-Leu-NH_{2 **3**}
Leu-Lys-**Leu**-Leu-Lys-**Lys**-Leu-**Leu-Lys**-Lys-Leu-Leu-**Lys**-Leu-Leu-NH_{2 **4**}
Lys-Leu-**Leu**-Leu-Arg-**Leu**-Leu-**Lys-Lys**-Leu-Leu-Arg-**Leu**-Leu-Lys-NH_{2 **6**}
Lys-Leu-**Leu**-Lys-Leu-Leu-**Lys-Lys**-Leu-**Leu**-Lys-Leu-**Leu**-Leu-Lys-NH_{2 **7**}
Gly-Leu-**Leu**-Leu-Arg-**Leu**-Leu-Lys-**Lys-Lys**-Leu-Arg-Leu-**Leu**-Lys-Lys-NH_{2 **5**}
Gly-Leu-**Leu**-Lys-Leu-Leu-**Lys-Lys**-Leu-**Leu**-Lys-Leu-**Leu**-Leu-Lys-Lys-NH_{2 **8**}

15. Composition pharmaceutique selon la revendication 13 ou 14, pour le traitement des infections provoquées par des bactéries résistantes aux antibiotiques comprenant un peptide diastéréomère choisi parmi les peptides de séquences :
Lys-Leu-**Leu**-Leu-Lys-Leu-Leu-**Lys-Lys**-Leu-Leu-Lys-**Leu**-Len-Lys-NH_{2 **2**}
Lys-Leu-**Leu**-Leu-Arg-**Leu**-Leu-**Lys-Lys**-Leu-Leu-Arg-**Leu**-Leu-Lys-NH_{2 **6**}

16. Composition pharmaceutique selon la revendication 13, pour le traitement des infections fongiques comprenant un peptide diastéréomère choisi parmi les peptides de séquences :
Lys-Leu-**Leu**-Leu-Lys-Leu-Leu-**Lys-Lys**-Leu-Leu-Lys-**Leu-**Leu-Lys-NH_{2 **2**}
Leu-Lys-**Leu**-Leu-Lys-Lys-Leu-**Leu-Lys**-Lys-Leu-Leu-**Lys**-Leu-Leu-NH_{2 **3**}
Leu-Lys-**Leu**-Leu-Lys-**Lys**-Leu-**Leu-Lys**-Lys-Leu-Leu-**Lys**-Leu-Leu-NH_{2 **4**}
Lys-Leu-**Leu**-Leu-Arg-**Leu**-Leu-**Lys-Lys**-Leu-Leu-Arg-**Leu**-Leu-Lys-NH_{2 **6**}
Lys-Leu-**Leu**-Lys-Leu-Leu-**Lys-Lys**-Leu-**Leu**-Lys-Leu-**Leu**-Leu-Lys-NH_{2 **7**}
Gly-Leu-**Leu**-Leu-Arg-**Leu**-Leu-**Lys-Lys**-Leu-Leu-Arg-**Leu**-Leu-Lys-Lys-NH_{2 **5**}
Gly-Leu-**Leu**-Lys-Leu-Leu-**Lys-Lys**-Leu-**Leu**-Lys-Leu-**Leu**-Leu-Lys-Lys-NH_{2 **8**}

17. Composition pharmaceutique selon l'une quelconque des revendications 11 à 16, sous la forme de solution, dispersion colloïdale, crème, lotion, gel, mousse, spray, aérosol ou autre formulation pour application nasale ou pulmonaire.

18. Composition pharmaceutique selon la revendication 11, pour le traitement du cancer.

19. Composition pharmaceutique anticancéreuse selon la revendication 18, comprenant un peptide diastéréomère choisi parmi les peptides de séquences :
Lys-Leu-**Leu**-Leu-Lys-Leu-Leu-**Lys-Lys**-Leu-Leu-Lys-**Leu**-Leu-Lys-NH_{2 **2**}
Gly-Leu-**Leu**-Lys-Leu-Leu-**Lys-Lys**-Leu-**Leu**-Lys-Leu-**Leu**-Leu-Lys-Lys-NH_{2 **8**}
Lys-Leu-**Leu**-Arg-Leu-Leu-**Lys-Lys**-Leu-**Leu**-Arg-Leu-**Leu**-Leu-Lys-NH_{2 **9**}
Lys-Leu-**Leu**-Leu-Lys-**Leu**-Leu-**Lys-Lys**-Leu-Leu-Lys-**Leu**-Leu-Lys-NH_{2 **10**}

20. Composition pharmaceutique selon la revendication 18 ou 19, pour le traitement de mélanomes, des carcinomes, basocellulaire et spinocellulaire, ou destinée au traitement des tumeurs du sein, du colon, du poumon ou de la prostate.

21. Composition pharmaceutique selon la revendication 20; pour le traitement des mélanomes du sein, du colon, du poumon ou des tumeurs de la prostate, comprenant un peptide diastéréomère choisi parmi les peptides de séquences :
Lys-Leu-**Leu**-Arg-Leu-Leu-**Lys-Lys**-Leu-**Leu**-Arg-Leu-**Leu**-Leu-Lys-NH_{2 **9**}
Lys-Leu-**Leu**-Leu-Lys-**Leu**-Leu-**Lys-Lys**-Leu-Leu-Lys-**Leu-**Leu-Lys-NH_{2 **10**}

22. Composition comprenant un peptide diastéréomère selon l'une quelconque des revendications 1 à 10, destinée à contrôler l'infection mycoplasmique dans une culture cellulaire, pour la conservation des aliments ou pour une utilisation en tant que complément alimentaire.

23. Composition vétérinaire comprenant un peptide diastéréomère selon l'une quelconque des revendications 1 à 10.
